(19) Europäisches Patentamt
European Patent Office
Office européen des brevets

(11) Publication number : **0 470 323 A2**

(12) **EUROPEAN PATENT APPLICATION**

(21) Application number : **90830392.8**

(22) Date of filing : **06.09.90**

(51) Int. Cl.$^5$ : **C07D 213/80,** C07D 295/108, A61K 31/465

(30) Priority : **30.07.90 IT 4817990**

(43) Date of publication of application :
**12.02.92 Bulletin 92/07**

(84) Designated Contracting States :
**AT BE CH DE DK ES FR GB GR LI LU NL SE**

(71) Applicant : **BROMLEY LIMITED**
**Cento Chalk Road**
**London NW1 8EH (GB)**

(72) Inventor : **Giovannini, Massimo**
**Via Francesco Coletti, 22**
**Roma (IT)**

(74) Representative : **Gristina, Giorgio**
**Studio Rag. GRISTINA Giorgio Via Po, 49**
**I-00198 Roma (IT)**

# (54) New salts of the nicotinic acid with aminobenzylic compounds and their pharmaceutical use.

(57) The instant invention is referring to some new salts of the nicotinic acid with some aminobenzylic compounds having a remarkable activity as hypolipaemizing, antispastic, vessel dilating and antiaggregating materials.

EP 0 470 323 A2

Jouve, 18, rue Saint-Denis, 75001 PARIS

The instant invention is referring to some new salts of the nicotinic acid with aminobenzylic compounds, and their therapeutical use.

At the state of the art, there are already some aminobenzylic compounds having a therapeutical activity. As an example, since some dozens of years it is known that some aminoketones have their ketone bound to a benzylic group and their addition salts with some no toxic acids; there are also many addition salts with organic and inorganic acids. These compounds have, they say, substantially rheological properties, calcium-modulating qualities and anti-aggregating properties with an elective action on the peripheral microcirculation. The nicotinic acid is a vitamin being known since several dozens of years in treating the troubles of the lipidic metabolism, and also as a dilator at the level of small and medium vessels with therapeutically higher doses. Its main use, however, was oriented to the care and prevention of the lipidic metabolism troubles, since the indication of it as a dilating therapeutical agent at the level of the small and medium vessels has shown considerable limitations in the clinical practice. As a matter of facts, they think that the use of big doses of nicotinic acid as it is required in order to enlarge the small and medium vessels brings, as a consequence, some cases of blood "theft" to the damage of previously ischemic zones, where a part of the blood flow is furtherly taken away, this being due to the vessels dilating action of the above mentioned nicotinic acid.

Therefore, taking also the negative indications given by the clinical use of the nicotinic acid as a dilating agent of small and medium vessels into account in accordance with the above mentioned considerations, and by considering also the substantially different requirements for the therapy of patients with troubles in the microcirculation as well as contemporary hyperlipemia phaenomena, it was probably subject to a counter-indication to associate, this from a scientific point of view, the nicotinic acid with some compounds being substantially similar to those previously qualified as aminoketones or derivatives of it, when drugs are prepared. Moreover, among the great number of inorganic acids used to produce salts from the above compounds, a clear reference to the nicotinic acid use was never found. The only no-toxic and pharmaceutically tolerable acid used has been the chlorhydric acid. To the contrary of what one could have expected to find, now they did surprisingly find that the coupling of nicotinic acid with amino-benzylic compounds produces a therapeutically active agent, wherein the effects of aminobenzylic compounds whose activity was mainly vessels-dilating, anti-spastic and anti-aggregating with the nicotinic acid with its mainly hypolimizing activity, have a resulting mutual increase, with a remarkable synergism effect.

The salification of the nicotinic acid with the above mentioned compounds gives freedom to a molecule which, by putting at disposal of the organism only later the basic components, consents to enjoy the desired therapeutical effect by minimizing those which must be avoided.

The present invention therefore consists of new salts of the nicotinic acid with aminobenzyle compounds being represented hereafter by the general symbol I, wherein $R_1$, $R_2$, $R_3$, which can be absent or can represent equal or different groups: $C_1$-$C_5$ linear or branched-off alkyl group, or even the linear or

GENERAL FORMULA

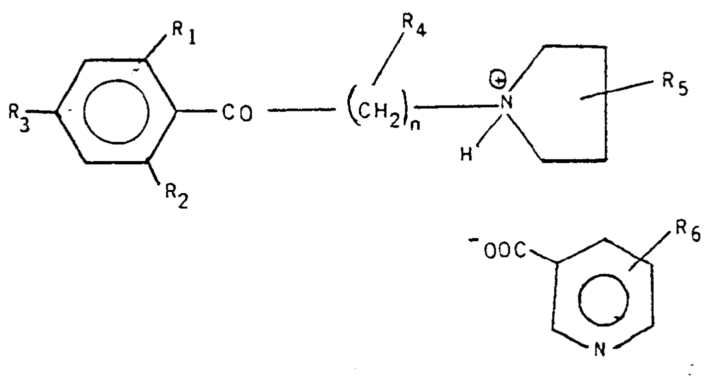

(I)

branched-off alkoxy $C_1$-$C_5$. "n" represents a whole number between 1 and 4; $R_4$ may be absent or may represent a linear or branched-off $C_1$-$C_5$ alkyl group, or even a halogen or a nitro-group; $R_5$ may be absent or may represent linear or branched-off $C_1$-$C_5$ alkyl groups or linear or branched-off alkoxygroups, or a halogen, or a nitrogroup; $R_6$ may be absent or may represent a linear or a branched-off $C_1$-$C_5$ alkyl group, or a linear or branched off $C_1$-$C_5$ alkoxy group, or a nitro group, or a halogen, or an identical group.

The instant invention consists also of a blood fats reducing agent, an anti-spastic agent, a vessel dilating and anti-aggregating agent consisting of a compound whose formula is (I).

A further purpose of the instant invention is to make use of the compound whose formula is (I) when preparing medicines and drugs with anti-aggregating and vessels dilating, anti-spastic and blood fats reducing effect. A further and not the smallest purpose of the instant invention is to produce medicine compounds consisting of a therapeutically efficient quantity of the formula (I) compound plus a carrier or an excipient being pharmaceutically bearable.

The salts with the general formula I may be produced, for instance, in accordance with the here after described organic synthesis process, consisting of four steps wherein reference is made to a compound with general formula I, wherein "n" is equal to 3, $R_4$ and $R_5$ are not present and $R_1$, $R_2$, and $R_3$ are the same stuff and do represent methoxylic groups.

STEP 1

<u>Preparation of the 4-Chlorobutyrrilchloride</u>

A mixture of gammabutyrlactone(II) has been delivered together with an equivalent quantity of thionyl chloride in presence of some catalytical quantities of a Lewis acid, for instance zinc chloride until the reaction is substantially completed. The product obtained is 4-chlorobutyrrilchloride (III) and has been separated by distillation from the reaction mixture.

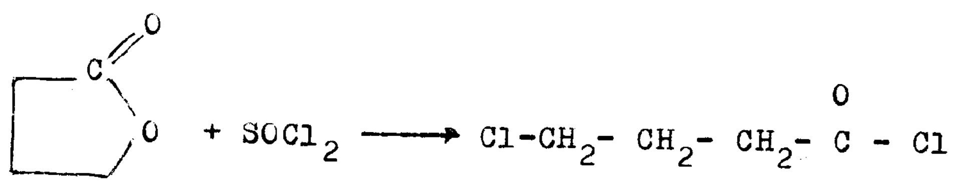

(II) (III)

STEP 2

Preparation of the 4-chloro-1,1-(2,4,6-trialkoxyphenyl)-1-butanone

A solution of 1,3,5-tri-alkoxybenzene being dissolved in methylene chloride ( a solvent for the Friedel Craft reactions) is added in a stirring state to an anhydrous suspension of anhydrous zinc chloride (one mole approximately) in the same solvent. The addition is made at temperatures between 0° to 30°C until same is completed. The mixture as it results from the reaction is cooled and at the same time one adds an aqueous solution of chlorhydric acid; the addition of the acid and the stirring continues until the Friedel Craft complex is hydrolized. The solution containing the product of the reaction (V) is washed in a diluted chlorhydric acid solution continuously, thereafter with a diluted sodium hydroxyde and finally with water. The solvent used to carry the product during the washing steps with aqueous solutions (methylene chlorhyde) can be removed at least partially before performing the following steps.

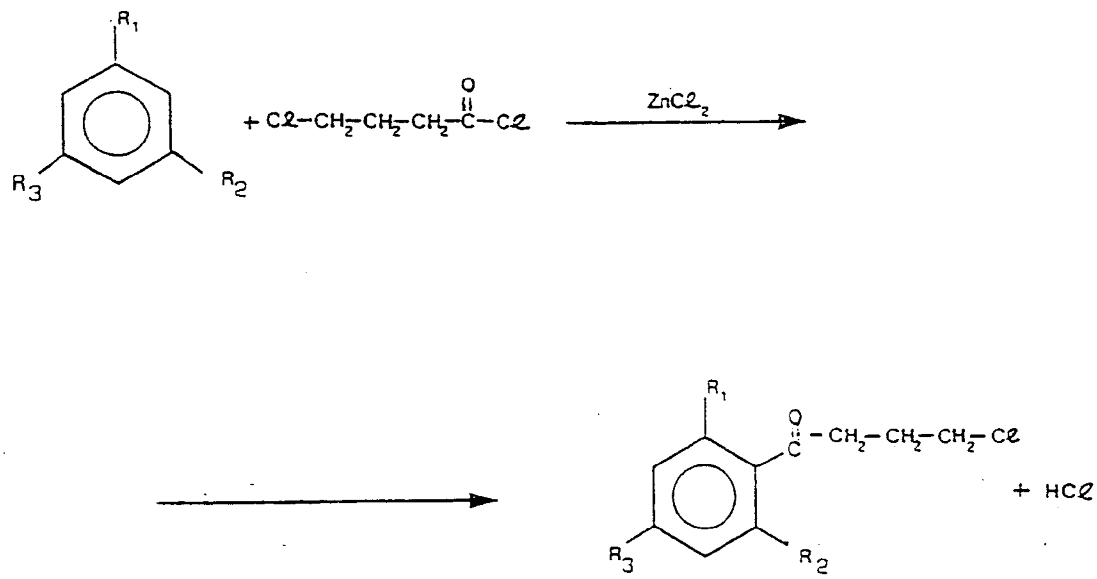


(V)

- Step 2

STEP 3

Preparation of the 4-pyrrolydil-1-(2,4,6-trialkoxyphenyl)-1-butanone

The preparation of the compound whose name is in the title is made by reaction between the compound of the formula (V) and the pyrrolydine used as an accepting substance of the hydrochloric acid being produced during condensation. The reagents must be heated to 50-100°C in a nitrogen area, in order to obtain that the reaction may be developed in an useful manner. The compound obtained has the formula (VII).

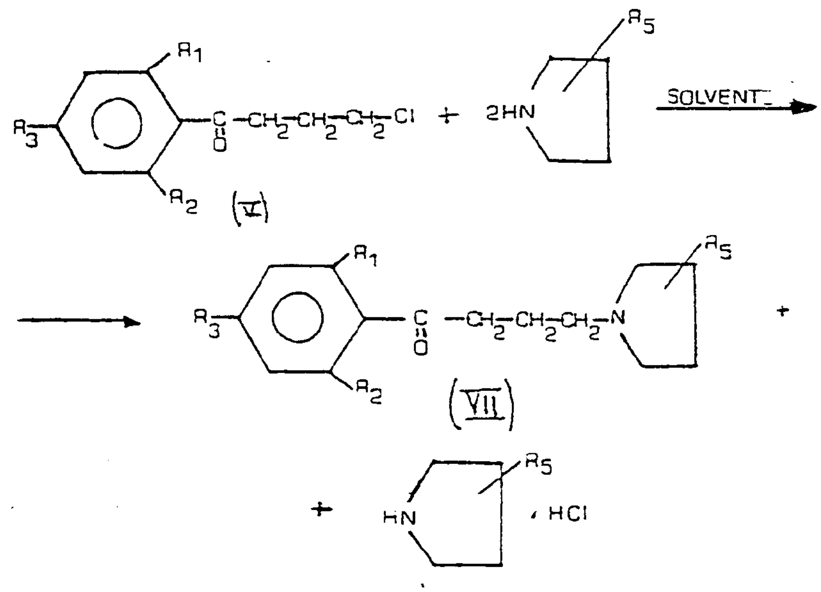


(V)

(VII)

STEP 4

Preparation of the 4-pyrrolidinyl-1,2,4,6-(3-alkoxyphenyl)-1-butanone nicotinized

The compound according to the formula (VII) is added with an equimolar quantity of nicotinic acid in pure ethanol solvent. A medium heating is performed, and thereafter one passes to the concentration under vacuum. The crystalline product obtained (VIII) is suspended in filtered ether by washing it with ether and it is recrystallized by pure ethanole/ether.

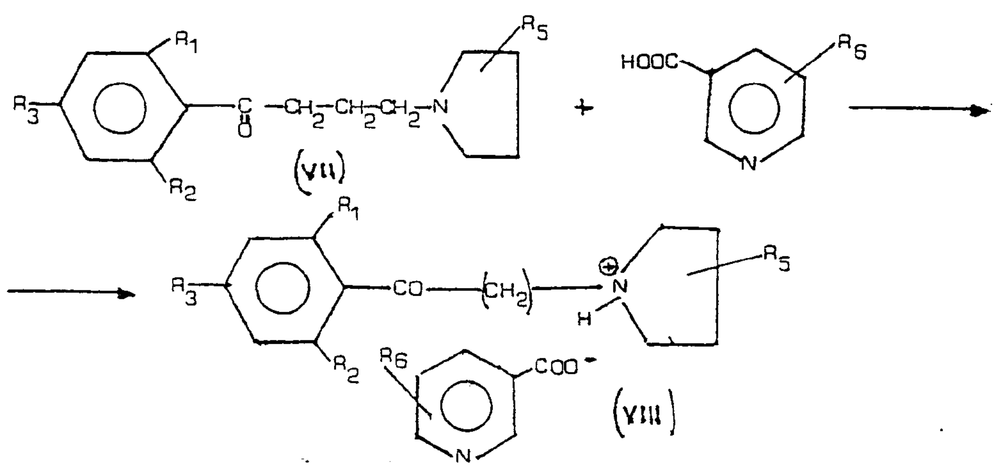

(VII)

(VIII)

Following the same proceeding as described in the steps 1 to 4, with various types of replace matters one did obtain various types of compounds with the general Formula (I) whereof in the following some examples are given:

Example 1: 4-(1 pyrrholydinyl)-1-(2,4,6 trimethoxy pheniyl)-1-nicotinized butanone

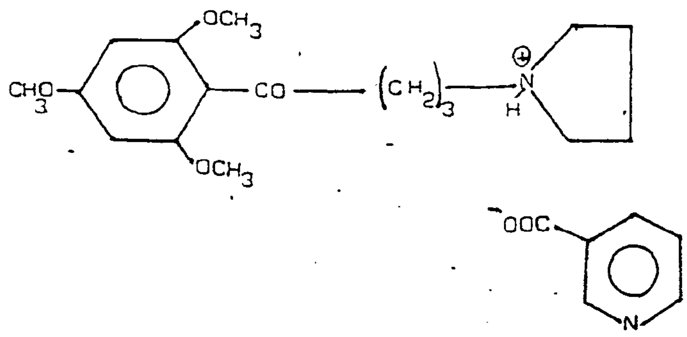

In the step 1 one has made use as compound (II) the γ-butyrolactone; in the step 2 as compound (IV) the 2,4,6 .tri-methoxyphenyl; in the step 3 one lets react the compound (V) resulting from the step 2, with the pyrrholydin thus obtaining the compound (VII). In the step 4 one lets to react (VII) with the nicotinic acid. In this way one obtains the compound according with the title.

Example 2: 4- (1-3 chloropyrrholydinyl)-1-(2,4,6 tri-methoxyphenyl)-1-nicotinized butanone

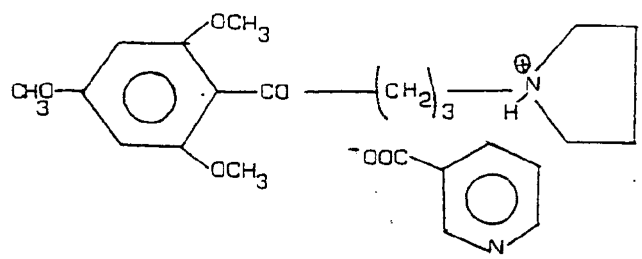

In the step 3 the compound is the 3-chloropyrrholydyn.

Example 3: 4-(1-pyrrholydinyl)-1-(2,4,6 tri-methoxyphenyl)-3-chloro-1-nicotinized butanone

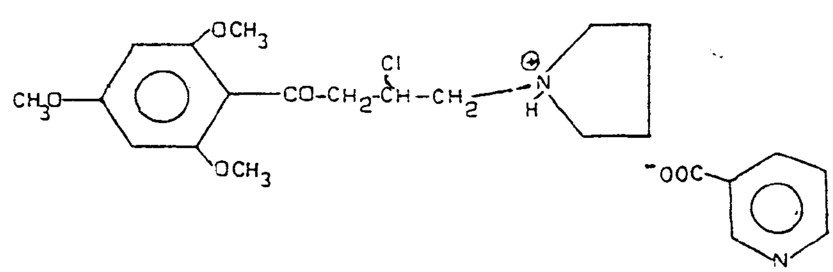

In the step 1 as compound (II) one makes use of the 3-chloro-gamma-butyrrholactone which gives place to the 3,4 dichloro butylchloride

Example 4: 4-(1-Pyrrhodinyl)-1-(2,4,6 triethoxyphenyl)-1 nicotinized butanone

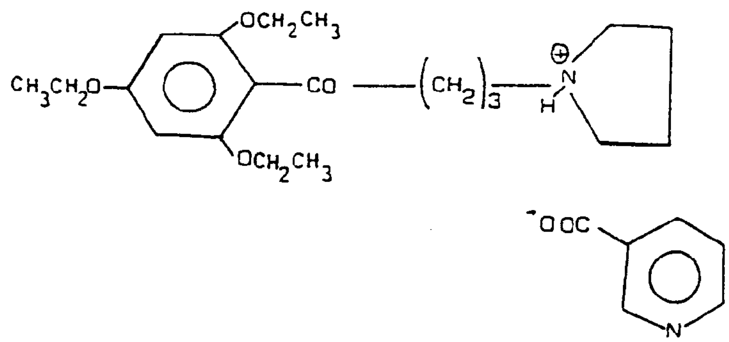

In the step 2 one makes use as compound (IV) of the 2,4,6 threeethoxyphenyl.

Example 5: 4-(1-pyrrholydinyl)-1-(2,4,6 tri-methoxyphenyl)-1-nicotinized (n methylacethoxy) butanone

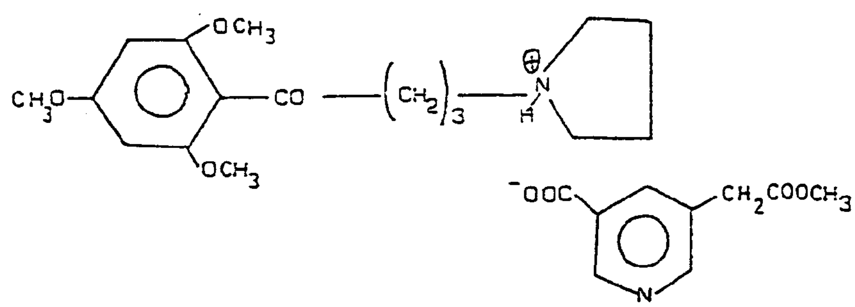

In the step 4 the compound having the formula (VII) is caused to react with the nicotinic acid replaced by half by the group $-CH_2COO.CH_3$.

Structural explanations performed for the compound according to the Example 1.

In order to confirm the structure for the compound according to the example 1, the following researches have been made:

1) NMR $C^{13H}$

2) Spectroscopy NMR $H^1$ (of the compound according to the example 1).

1) NMR $C^{13}$

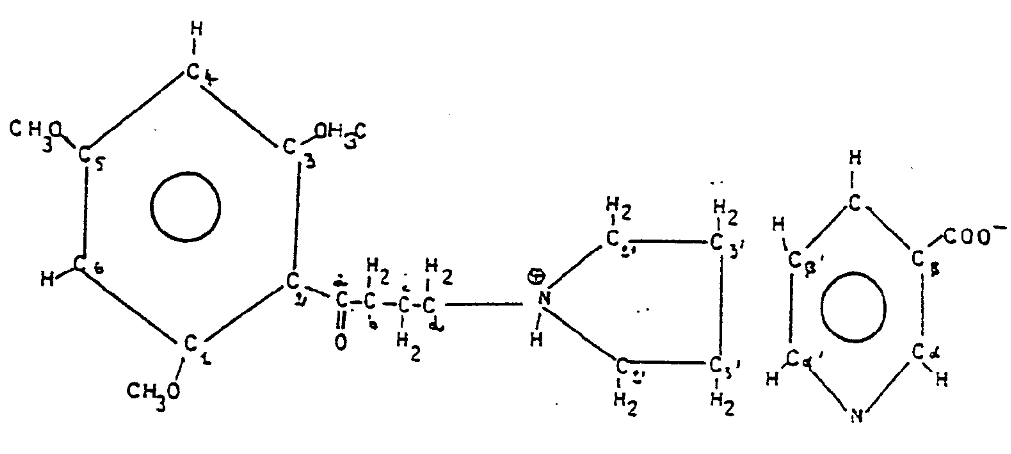

| ppm | frequency | attribution |
|---|---|---|
| 21 | 532,52 | "c" carbon (central $CH_2$ alkyl chain) |
| 23.5 | 591.13 | "3'" carbon pyrrholydinic ring |
| 41,6 | 1047,03 | "b" carbon (alkylic chain) |
| 54,9 | 1379,88 | signal caused by the carbon of the $-OCH_3$ |
| 56,3 | 1381,28 | "d" carbon (alkylic chain) |
| 56,62 | 1124,73 | "2" carbon of the pyrrholydinic ring |
| 91,857 | 2311,11 | Carbons of the aromatic ring whose names are "3" and "5" |
| 112 | 2822,46 | "C" carbon of the aromatic ring |
| 124,7 | 3137,36 | "$\beta'$" carbon of the nicotinic ring |
| 133,3 | 3354,53 | "$\beta$" carbon of the nicotinc ring |
| 138,5 | 3486,90 | "$\gamma$" carbon (nicotinic ring) |
| 149,7 | 3767,66 | "$\alpha'$" carbon (nicotinic ring) |
| 150,8 | 3795,03 | "$\alpha$" carbon (nicotinic ring) |
| 159 | 4007,19 | Carbons (2 and 6 of the aromatic ring |
| 163,7 | 4118,79 | "4" carbon aromatic ring |
| 173,09 | 4355,14 | Carboxylic carbons both |
| 207.08 | 5210 | |

2) NMR - $H^1$

| Position in th.molecule | Chemical shift (ppm) | Multiplicity (J in $H_2$) | Number of the $H_2$ atoms |
|---|---|---|---|
| 2 | 2,10 | m | 2 |
| 3 | 2,10 | m | 2 |
| 3' | 2,10 | m | 2 |
| 4' | 2,10 | m | 2 |
| 4 | 2,98 | +(J=6,8) | 2 |
| 2'a,5'a | 3,22 | t(J=8,0) | 2 |
| 2'b,5'b | 3,22 | dd (j=5,3 11,0) | 2 |
| O-OMe | 3,86 | S | 6 |
| p-oMe | 3,90 | S | 3 |

| | | | |
|---|---|---|---|
| 3" 5" | 6,35 | 5 | 2 |
| Nicotinized | | | |
| 5 | 7.53 | dd (j=7,8,5,0) | 1 |
| 4 | 8,26 | dt (J=7,8,2,0,2,0) | 1 |
| 6 | 8,62 | dd (j=5,0,2,0,2,0) | 1 |
| 2 | 8,96 | d (j=2,0) | 1 |

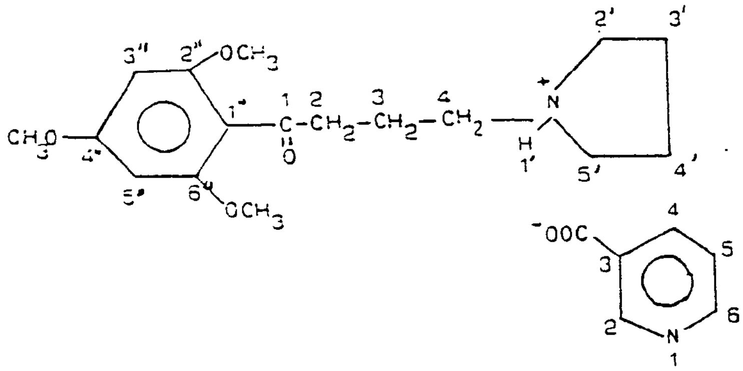

Spectroscopy IR (on the compound according to the example 1)

| Wavelength ($cm^{-1}$) | Attribution |
|---|---|
| 2840 | aromatic $-OCH_3$ |
| 2500 / 2700 | streching $NH^+$ |
| 1700 | stretching ketone C-Ar |
| 1610 - 1550 | ion COO- |
| 1400 | asymmetric and symmetric stretching |
| 1220 - 1275 | C-O-C stretching aromatic ethers |
| 1715 - 1125 | 1,3,5 threereplaced aromatic rings |
| 1070 - 1000 | |

MASS SPECTROSCOPY

(on the compound according to the Ex.1)

| M/e | Structure | rel. abundance, % |
|---|---|---|
| 97 | 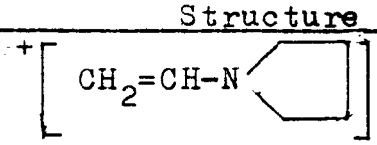 | 100 |

84 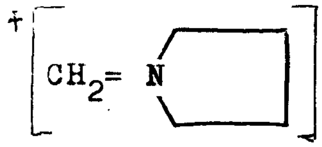 80,3

123 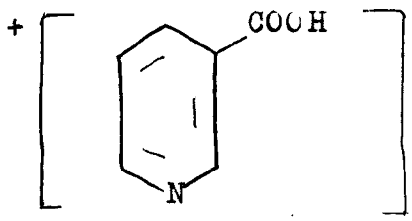 49,21

195 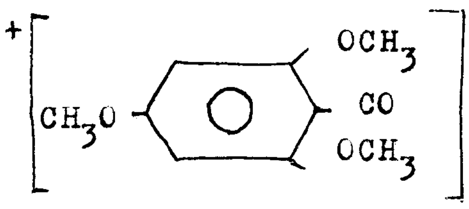 30,93

307 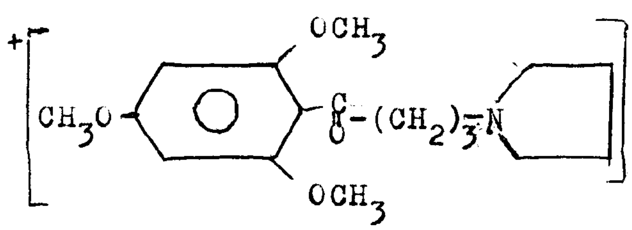 21.45

78 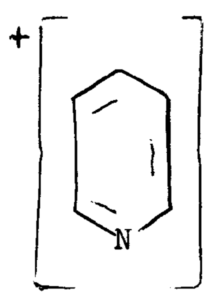 18.19

210 + 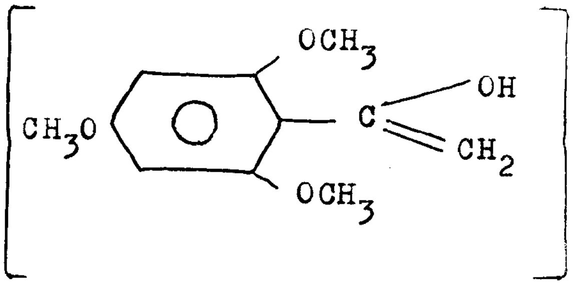 10.93

Elemental Analysis (on the compound according to the Example 1)

| | Carbon % | Nitrogen % | Hydrogen % |
|---|---|---|---|
| Reckoned | 64.07 | 6.51 | 7.02 |
| Found | 63.92 | 6.31 | 6.92 |
| | 64.00 | 6.50 | 7.01 |
| | 64.10 | 6.55 | 7.05 |
| | 64.07 | 6.49 | 7.10 |
| | 63.98 | 6.48 | 7.06 |
| X | 64.01 | 6.47 | 7.04 |
| s.d. | 0.07 | 0.09 | 0.05 |

In form of examples, here one may find some preferred formulations of the compound with general formula (1) in form of tablets, injection vials or drops.

In form of examples, here one may find some preferred formulations of the compound with general formula (1) in form of tablets, injection vials or drops.

Gastro-soluble tablets (standard quantity for 1000 tablets).

Compounds according to the example 1: 375.5 g
Lactose EP: 40 g
Maize starch F.U.: 16.0 g
Stearate magnesium N.F.: 2,5 g
De-ionized water F.U.: the required quantity for the granules.
Methocel white covering solution (with a low TiO2 percentage 105,0 ml
Polyethylenglycol 8000 NF powder: 0.5 g

Drops (quantity for 1000 ml)

Compound according to the example 1: 188,0 g
Propylenic glicol: 100,0 ml
Sodium benzoate F.U.: 15,0 g
Glycirrhizine ammonium: 50,0 g
Aroma: 60,0 g
Saccharin with Na USP: 1,0 g
De-ionized water USP q.suff. to 1000ml

Gastro-resistant tablets (quantities for 1000 tablets)

The compound according to the Example 1 in the same quantity as for the gastro-soluble tablets

The gastro-resistant coating is, as an example, as follows:

Eudagit L: 18,8 g
EG 6000 FU : 1,8 g
Dibutylphtalate: 0,5 g
Di-methicone 200 FU : 0,3 g
Talc FU: 3,6 g

Mutagenetic effects

The compound according to the Example 1 has been tested, in order to check and see its mutagenic effects. The effects on five Thyphirium salmonella strains (TA 98, TA 100, TA 1535, and TA 1538) treated with the compound according to the Example 1, did not show any mutagenic activity both with and without the presence of the microsomial fraction of rat liver at the different quantites as submitted. Moreover the same salty compound did not increase, with the tested dosages, the frequency of mutations in the pl strain of Schiosaccharomyces Pombe with and without the presence of the microsmial fraction. Moreover, the treatment of HeLa human cells cultures with the compound of the example 1 did not determine any variation, with reference to the controls treated with solvent, in the quantity of tritium-added Thymidine being included into the DNA of the cells as a confirmation that there is no repair synthese (not being programmed by the DNA at the dosages being tested).

Moreover, when one uses the same material as such with metabolic activation, the urines of the rats having been treated by intragastric way with two doses of the compound according to the example 1, any beta-glucoronidase being absent, any mutagenic action did not result, on the five strains of Typhumurium Salmonella as above reported, on the Schiosaccharomyces Pombe and on the HeLa cells (DnA-repair).

During these pharmacologic tests, the effect in the compound according to the example 1 has been shown.

Pharmacological and clinical effects

The results of the pharmacological and clinical tests have been achieved with the compound in accordance with the example 1.

The product according to the Example 1 did show the following picture of pharmacological and clinical properties.

Effects of the hypertri-glyceridesaemia from olive oil in the rat

The method described by Zucker I.H. (Sci. Anal. Lab. 24, 114, 1972) has been followed.

Male rats have been used, whose body weight was 150-200 g. The animals were without eating with water "ad libitum" and all this beginning 24 hours before the experiment. The hyperlipaemia was induced by the administration of olive oil by mouth; its composition as to the fat acids was as follows: palmitic acid 10% palmitoleic acid 1%, stearic acid 3%, linoleic acid 80% and linoleic acid 7% (all percentages are by weight). The quantity of olive oil administered, by means of a gastric probe, was 10 ml/kg. The treatments were made by finely pounding with a china mortar and by suspending in the olive oil the examined compounds. The compounds according to the example 1 is not present. The positive control animals received only olive oil; moreover, there was also a group of negative control animals who received plain water in quantities of 10 ml/kg. Every three hours a group of rats (10) was killed in a light ethereal anaesthesia, in order to be able to appreciate the percentage of triglycerides in the blood. The animals were bled by cutting them the carotids and the blood was collected in plastic test-tubes, capacity 10 ml each, and was centrifuged for ten minutes at 3500 rpm. The evaluated parameter was the lactescence of the serum, which is directly proportional to the contents of kilomicrons and therefore of triglycerides (Zucker, see above the mention). The serum has been diluted 1:5 in a physiologic solution and was read at the spectrophotometer at 525 mm against white (physiologic solution). From the individual optical Density (D.O.) data one could calculate for each group the average of the standard error. The statistical analysis was performed by means of the t of Dunnett. The results are given in the Table N°1. In the negative control animals, i.e. those who did not receive the olive oil, one may see an optical density (D.O.) of the serum very low and the same is decreasing in the course of the experiment. In the positive control animals the optical density on the contrary was considerably increasing and it arrived to very high values, seven times approximately higher than the void control animals. The treatment with nicotinic acid in quantities of 2 mM/kg did reduce, even if not very much, the optical density of the serum. The treatment with the compound

according to the example 1, already at the dosage of 1 mM/kg, did have an effect scarcely above that of the nicotinic acid 2 mM/kg. At the dosages 2 and 4 mM/kg the activity of the compound according to the example 1 did continuously increase. The mixture of nicotinic acid and Buflomedil had practically the same induced effect of the nicotinic acid at the same dosage. (Table 1)

Table N°1
Hypolipaemizing activities
Effect in the hypertrigliceridaemia from olive oil in the rats

| Treatment | Dose mM/kg/os | N° anim. | Body wt M ± E.S. | Serum opt. density at the indicated times (M ± E.S.) 3h | 6h | 9h | 12h | 16h | 18h |
|---|---|---|---|---|---|---|---|---|---|
| Negative controls | ... | 10 | 203 ± 3,41 | 217 ± 15,4 | 234 ± 14,7 | 226 ± 16,3 | 212 ± 14,1 | 207 ± 16,7 | 197 ± 15,9 |
| Positive controls | ... | 10 | 207 ± 3,26 | 263 ± 16,2 | 1617 ± 66,3 | 1721 ± 54,1 | 1356 ± 33,1 | 722 ± 29,6 | 310 ± 20,3 |
| Nicotinic acid | 2 | 10 | 212 ± 3,00 | 258 ± 14,9 | 1312 ± 68,2 * | 1161 ± 63,2 * | 1000 ± 31,3 * | 610 ± 18,4 * | 250 ± 21,4 |
| | 1 | 10 | 210 ± 3,212 | 217 ± 16,3 | 1121 ± 61,6 * | 987 ± 36,7 ** | 724 ± 28,4 ** | 458 ± 21,3 * | 229 ± 22,6 |
| Compound according to Example N°1 | 2 | 10 | 206 ± 2,982 | 271 ± 16,9 | 856 ± 44,2 ** | 624 ± 27,4 ** | 451 ± 26,2 ** | 294 ± 26,2 ** | 212 ± 28,3 * |
| | 4 | 10 | 209 ± 2,742 | 260 ± 17,1 | 720 ± 43,8 ** | 612 ± 21,2 ** | 400 ± 28,3 ** | 226 ± 25,6 ** | 184 ± 16,2 * |
| Buflomedil +Nicotinic acid | 2+ 2 | 10 | 216 ± 2,732 | 256 ± 14,9 | 1315 ± 35,1 * | 1156 ± 38,3 * | 1021 ± 39,2 * | 601 ± 33,7 * | 340 ± 21,3 |

* $P < 0,05$ ** $P < 0,01$, vs. positive tests (Dunnett test)

Hypercholesterolaemia of the mouse, caused by Triton WR 1339

The method described by Garattini S. (Arzneim,Forsch 9, 206; 1959) with some modifications has been followed.

The mouses used have been males of 22 to 28 grams body weight, on an empty stomach but with water "ad libitum" since 12 hours. The hyperlipidaemia was induced by means of an intraperitoneum administration of 10 mg/kg of Triton WR 1339, with dosage 300 mg/kg in a physiological solution. The subject products were administered at the same time in clear plain water, always in a volume of 10 mg/kg by means of a gastric probe. The animals were sacrified after 20 hours, by cutting them the carotids. The blood was collected in some plastic test-tubes, and was centrifuged at 3500 rpm for ten minutes. Thereafter the total cholesterol (Sornay R and colleagues, Arzeneim Forsch, 26,885 1976) and the total lipids (Chonhelmer R and colleagues, Biological Chemistry 106, 745, 1934) were determined. In order to be able to correctly evaluate the results, for each group we reckoned the average, and the standard error; tire significance with the animals taken as controls was obtained with the Dunnett test (Biometrics 20, 482; 1964). The insults of these tests are given in the table N°2. In the hyperlipaemic controls with the Triton WR 1339 injections and with a dosage of 30 mg/kg i.p. the cholesterolaemia was as an average 358 mg/1000 ml and the lipidaemia 2436 mg/100 ml totally. In the animals treated by means of nicotinic acid and dosage 2 mM/kg weight it is possible to remark respectively a reduction of a 39,9 % and 32,5 %. With the compound according to the example 1, already at a dosage of 1 mM/kg weight, the reduction was 38,3% for the cholesterol and 29,3°% for

Table N° 2
Hypercholesterolaemia caused by Trit WR1339 in the mouses

| Treatment | Dose mM/kg per os | n° animals | Body wt grams M ± E.S. | Tot.Cholesterol mg/100 ml |  | Tot.Lipids mg/100 ml |  |
|---|---|---|---|---|---|---|---|
|  |  |  |  | M ± E.S. | % vs contr. | M ± E.S. | % vs contr. |
| Controls | --- | 10 | 25,2 ± 0,31 | 358 ± 22 | -- | 2436 ± 242 | --- |
| nicotinic acid | 2 | 10 | 26,4 ± 0,67 | 215 ± 16 ** | 39,9 | 1643 ± 131 * | 32,5 |
| Compound Example 1 | 1 | 10 | 24,9 ± 0,51 | 221 ± 18 ** | 38,3 | 1721 ± 164 * | 29,3 |
|  | 2 | 10 | 25,0 ± 0,42 | 191 ± 15 ** | 46,6 | 1315 ± 115 ** | 46,0 |
|  | 4 | 10 | 26,2 ± 0,47 | 133 ± 12 ** | 62,8 | 1008 ± 117 ** | 58,6 |

* $P < 0.05$ ** $P < 0.01$ vs. Controls (Dunnett test)

the lipids totally. At the dosage of 2 mM/kg weight, the compound according to the example 1 causes a reduction in both parameters of 43% whereas at the dosage of 5 mM/kg weight the reduction is 60 % approximately.

Even in this case, in equimolar quantities, the activity of the compound according to the example 1 is above that of the nicotinic acid.

Effects of the hypertriglyceridaemia caused by ethanole in the rats.

For this test group the methods described by Sistori C.R. and colleagues (Atherosclerosis 30, 45; 1978) is followed.

The hypertriglyceridaemia has been induced into the male rats having a body weight of 160 - 170 g by giving them to drink, for 4 consecutive days, a solution of 10% ethanole in water. Moreover at the evening of the third day and at the morning of the fourth day, each animal has received, by means of a gastric probe, 0,5 g of ethanole in 2 ml of fresh water; some animals in groups have been kept as negative controls. The treatment with the compound according to the example 1 and with nicotinic acid has been performed once a day in the four days when the ethanole was administered.

By using the above described methods, in two different tests both the activity of the compound according to the example 1, at three dosage levels, and also the duration of the effect of a dose level have been evaluated.

Activity at three dose levels

The effectiveness of 0,5-1-2 mM/kg of the compound according to the example 1 has been compared to that of 1 mM/kg "per os" of the nicotinic acid in groups of ten rats sacrified four hours after the last treatment.

The results are reported in the Table 3.

Table N° 3

Hypertriglyceridaemia caused by ethanole in the rats. Effects 4 hours after treatment.

| Treatment | Dose mM/kg/os | n° animals | Triglycerids mg/100 ml M ± E.S. | Triglycerids mg/100 ml % vs contr. positivi | Meaning against positive controls * |
|---|---|---|---|---|---|
| Negative controls | --- | 10 | 111,3 ± 14,6 | -45 | --- |
| Positive controls | --- | 10 | 202,4 ± 19,3 | --- | --- |
| Nicotinic acid | 1,0 | 10 | 156,4 ± 18,1 | -24,7 | < 0,05 |
| Compound of the Example 1 | 0,5 | 10 | 148,2 ± 16,4 | -26,8 | < 0,05 |
| | 1,0 | 10 | 135,0 ± 12,6 | -33,6 | < 0,01 |
| | 2,0 | 10 | 112,6 ± 14,3 | -44,4 | < 0,01 |

* Dunnett test

In the negative control animals (normal animals) the hypertrigliceridaemia is of 111,3 mg/100 ml. In those having been treated with ethanole (positive controls) it is increased to 202,4 ± 19,3 mg/100 ml. The nicotinic acid at the dose of 1 mM/kg per os produces a statistically meaningful decrease of 24,7%; the treatment with the compound according to the example 1 at doses of 0,4 - 1 - 2 mM/kg determines respectively decreases of 26,8% - 33,3% and 44,4% all being very meaningful from a statistical point of view (Table 3).

In order to perform the particular kinetics of the compound in accordance with the example 1 following the instant invention, it was considered appropriate, anyway, to evaluate the duration of the hypolipaemizing effect in comparison to that of the nicotinic acid.

Table N°4

Hypertriglyceridaemia caused by ethanole in the rats. Duration of the effects.

| Treatment | Dose mM/kg/os | n° animals | Trigliceridi (mg/100 ml) 4h | 6h | 12h | 16h | 24h |
|---|---|---|---|---|---|---|---|
| Negative controls | --- | 10 | 131,3 ± 10,4 | 124,2 ± 11,2 | 128,7 ± 11,6 | 121,9 ± 9,3 | 123,2 ± 9,6 |
| Positive controls | --- | 10 | 257,2 ± 23,8 | 236,3 ± 27,1 | 205,8 ± 16,8 | 186,5 ± 14,7 | 165,3 ± 10,5 |
| Nicotinic acid | 1 | 10 | 181,6 ± 15,7 • (-29,4) | 176,0 ± 11,2 • (-25,5) | 184,6 ± 8,9 (-10,3) | 180,7 ± 14,5 (-3,1) | 178,4 ± 9,7 (+7,9) |
| Compound of Ex. 1 | 1 | 10 | 153,9 ± 11,6 •• (-38,2) | 135,6 ± 10,9 •• (-42,6) | 141,0 ± 21,1 • (-31,5) | 145,3 ± 12,7 • (-22,1) | 150,1 ± 8,4 (-9,2) |

In parenthesis : % variation vs positive controls

• $P < 0{,}05$ •• $P < 0{,}01$ vs positive controls (Dunnett test)

Groups of ten rats having received ethylic alcohol as it was made previously, have been treat ed "per os" with a vehicle (positive control rats), nicotinic acid (1 mM/kg) or with the compound in accordance with the example 1 (1 mM/kg) and killed at distances of 4 - 6 - 12 - 16 and 14 hours from the last treatment. A group of normal animals was treated with the vehicle only and did serve as a negative control group.

The results are reported in the Table N°4.

In the normal animals the average concentration of triglycerides in the serum remains unchanged for all the duration of the test, at around 120 - 130 mg/dl.

In the positive control animals, treated with ethylic alcohol, an hyperglyceridaemia is found, with a duration of 24 hours and slowly fading from the twelfth hour on. The administration of 1 mM/kg of nicotinic acid caused a very significant reduction of the silk triglycerides with dosages after 4 and 6 hours from the last treatment. After such a period the concentration of the triglycerides does not result lower in a significant measure to that of the positive control animals. On the contrary the compound according to the example 1 and with equimolar dosage, did show to have a stronger and more durable activity. The effect, at its maximum at the sixth hour of the treatment, is still evident at the 16th hour.

CARDIOCIRCULATORY ACTIVITY

Antispastic activity in the central artery of the ear in the rabbit.

An "in vitro" method has been used, according to which the withdrawal of the central artery of a rabbit's ear with the relevant nervous connections and the equipment of an appropriate seat for secluded organs was designed (Fleckenstein A., British Journal of Pharmacology 7, 533, 1952).

On the artery they did install a three-ways tube, connected with a peristaltic pump and with a mercury manometer to record the perfusion pressure. The artery was perfused with a rebs liquid at the speed of 2,5 ml/minute. The contractions were induced by injecting into the vessel 0,1 ml adrenalin at the concentration of 1 mcg/ml. The compound according to the example 1 has been added to the bath at concentrations of 0,5 - 1 - 2 uM, five minutes before the adrenalin (four preparations per concentration). As comparison drugs, chlorhydra ted Buflomedil and Papaverin have been used. The activity of the material under investigation has been appreciated in terms of percentages referring to the concentration induced by the Adrenalin (and expressed with $DE_{50}$) and the relevant forbidding.

TABLE N° 5

"in vitro" antispastic action. Central artery of a rab bit's ear. Spasm induced by Adrenalin (n=4)

| Treatment | μM Concentration | % inhibitions of the contractions | DE50 (μM) | Reliance limits 95% |
|---|---|---|---|---|
| Papaverin | 0,5 | 33 | | 1,67 |
| | 1,0 | 64 | 0,76 | |
| | 2,0 | 81 | | 0,34 |
| Buflomedil | 0,5 | 41 | | |
| | 1,0 | 60 | 0,67 | 1,49 |
| | 2,0 | 87 | | 0,31 |
| Compound ac | 0,5 | 45 | | |
| cord.Ex.1 | 1,0 | 70 | 0,58 | 1,18 |
| | 2,0 | 94 | | 0,29 |

As it's possible to see from the Table N°5, the compound according to the Example 1 performs a dose-depending antispasm action, a little above, in terms of DE50, over that of the Papaverina and of the Buflomedil.

Peripheral vesselsdilating activity in the mouse

The method of Richter W. (Acta Pharm. Toxicol. 21, 91; 1974) was used; it consists in determining, at fixed time intervals after the treatment, the skin temperature of the mouse's paw, by keeping the animals in rooms at a constant temperature of 21°C.

Some groups of ten male mouses each have been used; the weight of each animal was 30 g approximately.

The compound according to the Example 1 has been tested "per os" at three basic levels of the doses (0,125 - 0,25 - 0,5 mM/kg) and by intravenous injection at two dose levels (0,015 and 0,03 mM/kg), in comparison with one sole dose of Papaverin and chlorhydrated Buflomedil (0,5 mM/kg "per os" and 0,03m M/kg intravenous). The observation of the skin temperature at the mouse's paw was made before the treatment, 30, 60 and 120 minutes after the "per os" treatment, 10 - 30 and 60 minutes after the intravenous treatment by using a special thermocouple (A-HI Ellab) connected with an Ellab electric recorder. Of each animal the temperature of both hind paws has been measured, and the values obtained have been averaged .

From the data shown in the Tables N°6 and 7 it comes that the compound according to the example 1 administered "per os" has a more powerful and persisting vessels-dilating dose-depending activity than the one of the chlorhydrated Buflomedil. By intravenous way it results that the effects of both drugs can be superposed.

Anti-aggregating "ex vivo" activity against collagene in the rat

Six groups of 15 male rats of 200 - 250 g have been used.

Table N° 6

Peripheral vessels dilating activity in the mouse.

Temperature (°C) of the hind paws. Oral treatment.

| Treatment | Dose mM/kg/os | Minutes from the treatment 0 | 30 | 60 | 120 |
|---|---|---|---|---|---|
| Controls | — | 24,5 ± 0,25 | 24,3 ± 0,20 | 24,6 ± 0,30 | 24,4 ± 0,22 |
| Compound according to the Example 1 | 0,125 | 24,3 ± 0,24 | 24,7 ± 0,32 | 024,9 ± 0,30 | 25,2 ± 0,35 |
| | 0,250 | 24,7 ± 0,20 | 25,2 ± 0,44 | 25,8 ± 0,32 | 25,5 ± 0,50 |
| | 0,500 | 24,6 ± 0,25 | 26,8 ± 0,30 ** | 27,5 ± 0,40 ** | 27,2 ± 0,45 ** |
| buflomedil HCl | 0,500 | 24,2 ± 0,30 | 25,6 ± 0,25 * | 26,5 ± 0,50 ** | 26,1 ± 0,42 * |
| papaverin | 0,500 | 24,8 ± 0,35 | 25,5 ± 0,21 * | 25,9 ± 0,25 * | 25,1 ± 0,30 |

* $P < 0,05$ ** $P < 0,01$ vs controls (Dunnett test)

Table N°7

Peripheral vessels dilating activity in the mouse.

Temperature (°C) of the hind paws (n=10).

Intravenous treatment.

| Treatment | Dose mM/kg/iv | Minutes from the treatment 0 | 30 | 60 | 120 |
|---|---|---|---|---|---|
| Controls | — | 24,1 ± 0,35 | 24,4 ± 0,30 | 24,5 ± 0,32 | 24,3 ± 0,25 |
| Compound according to the Ex.1 | 0,015 | 24,3 ± 0,25 | 25,9 ± 0,18 ** | 25,8 ± 0,30 * | 25,4 ± 0,22 * |
| | 0,030 | 24,2 ± 0,20 | 25,7 ± 0,31 ** | 25,5 ± 0,27 ** | 25,3 ± 0,30 ** |
| buflomedil HCl | 0,030 | 24,3 ± 0,32 | 25,6 ± 0,20 ** | 25,3 ± 0,24 ** | 26,0 ± 0,18 ** |
| papaverina | 0,030 | 24,5 ± 0,28 | 25,6 ± 0,25 ** | 25,7 ± 0,20 * | 25,5 ± 0,23 * |

* $P < 0.05$ ** $P < 0.01$ vs controls (Dunnett test)

A group of animals has been treated with the vehicle only (control animals - 10 ml/kg per os of gum arabic, 5%) whereas the other groups with 0,25-0,5-1 mM/kg os of the compound according to the example 1, with nicotinic acid (1mM/kg os) or with Buflomedil 1mM/kg os. The treatment's duration has been 4 days. Two hours after the last treatment, three animals of each group, without eating since 18 hours and in ethereal anaesthesy, have been taken, and from the abdominal aorta were taken 4,5 ml of blood by means of the siliconed syringe containig 0,5 ml of sodium hepatin in phisiological solution (final concentration 50 I.U./ml of blood). The blood taken from the three rats of each group has been mixed and centrifuged during 6 minutes at 1400 rpm, at room temperature. After having taken the plasma full of platelets (PRP) the thereunder floating material has been centrifuged again (4000 rpm during 10 minutes) in order to have the plasma with few platelets (PPP) separated from the balance. The PRP has been diluted with a part of PPP in order to bring the number of platelets to 350 ± 50 x $10^3/mm^3$. The aggregation has been evaluated in accordance with the method of Minsker D.H. and coll. (J. Pharmacol.Exp.Ther. 210 37 1979) by using an ELVI 840 two-channels aggregometer and an Omniscribe recorder B.500. A quantity of 0,25 ml PRP has been used, transferred into the aggregometer for two minutes at 37°C and stirred at 1000 rpm. The light intensity was arranged in such a way, to obtain 0% transmittance with the PRP and 100% with the PPP. After 2′ incubation, by means of microsyringe the collagene was added to the PRP in a volume quantity of 5 mcl with the final concentration of 4 mcg/ml.

From the aggregation trace of the recorder the following data have been evaluated:

a) latent time of the aggregation (seconds between the introductions of collagene and the starting of the aggregation)

b) transmittance increase percentage (maximum aggregation). The same proceeding was repeated for all

animals, always by mixing the blood of three rats belonging to the same group and by obtaining in this manner five aggregation curves per group.

From the results given in the table N°8 one can observe that the administration of the compound according to the Example 1 causes a dose-depending decrease of the aggregation of the platelets caused by the collagene evaluated as latency time of the moment when the aggregation begins and as maximum aggregation (% of transmittance). The effect is significant beginning from 0,5 mM/kg os. The treatment with nicotinic acid does not modify in an evident way both parameters of the aggregation curves, whereas the effect ascertained after the administration of Buflomedil is approximately 50% of that caused by the same dose of the compound according to the example 1.

How the blood cells (red cells) of the rats may be deformed during the fitration possibility test

From the fonctional point of view, the grown-up blood cells have same important features about being deformed and this consents them to pass through capillary vessels having a diameter 20 and even 30% lower than their own diameter; in this manner they pass particularly well and at a certain distance between each other; this feature consents them to give to the surrounding space, in a particularly important and high manner, their oxygen load.

The perfect elasticity of the blood cells (red cells) the above being understood in a physical sense (Palmer A.A. Esp. Physiol. 44, 149, 1959) consents them to regain their discoid form without any elastic hy

Table N° 8

Anti-aggregating "ex vivo" activity against collagene in the rat.

| Treatment | | Latency time (sec.) | Transmittance percentage |
|---|---|---|---|
| Controls | --- | 37,5 ± 3,1 | 78,6 ± 5,8 |
| Compound according to the Example N° 1 | 0,25 | 43,4 ± 4,2 (+15.7) | 67,3 ± 4,4 (-14,4) |
| | 0,50 | 52,0 ± 3,5 * (+38,7%) | 51,6 ± 4,0 * (-34,3%) |
| | 1,00 | 64,5 ± 5,4 ** (+72,0%) | 30,2 ± 3,6 ** (-61,6%) |
| Nicotinic acid | 1,00 | 40,5 ± 4,0 (+8,0%) | 68,4 ± 5,2 (-13,0%) |
| Chlorhydrated Buflomedil | 1,00 | 51,9 ± 3,7 * (+38,4%) | 54,2 ± 4,5 * (-31%) |

*P < 0,05 **P < 0,01 vs controls (Dunnett test)

In parenthesis: % variation vs controls

steresis (Teiltel P.: Nouv. Rev. Franç. Hématol. 7, 195, 1967). Any alteration, almost undoubtely at the level of the red cell's membrane, cause some damaging variations of the red cell's elasticity thus making more difficult the passing through the capillary vessels having more reduced diameter, and therefore increasing the flow resistance in the peripheral vessels systems. In order to determine the effect caused by the compound according to the Example 1, on these features of the red cell, the method described by Schmidt, Schonbein and colleagues (Blut 26, 369, 1973) has been used.

In the circulatory flow, the above mentioned capillary vessels being excluded, the blood cells do normally circulate being attached between each other because of their adhesiveness. In order to study this adhesion force, the above mentioned Teitel method has been used. Male rats of 150 g body weight approximately have been employed, and they have been treated for three consecutive days with 0,5 mM/kg/os of the compound according to the Example 1, or otherwise Buflomedil, or even Pentosiphillin used as comparison substance. After the last treatment the animals, without eating from 18 hours, have been killed in a light ethereal anaesthesy and the blood was collected over an 0,2 molar EDTA (9 and 1 volumes respectively). The red cells filtering

possibility, measured with the Teitel method, wherein the quantity of red cells passing, in high haematocritical conditions, through a paper filter with holes of 20-40 µm in a previously determined time, has been ascertained as follows: the blood was centrifuged for ten minutes at 2000 rpm in order to obtain the massing of the red cells, two ml of the blood collected as above were put into an upside down turned cone made with filtering paper, being previously wetted with a physiological solution. The volume passing through the filter in 2 - 4 - 8 and 16 minutes is then measured. The average filtration time T 1/2 is reckoned on semi-logarithmic paper by putting on the abscissae the logarithm of the time, and on the ordinates the percentage of blood filtered. In order to follow the Schmidt Schönbein method, the here after described proceeding has been performed: 0,2 ml of a suspension of red cells at 2% obtained by diluting the blood with the autological plasma, then it was placed into an appropriate device permitting to filter this blood through a filter (millipores) of 5 µm mashes at a filtration pressure of 50 mm water. With a chronometer one determines the time required for the passage of the whole sample through the filter.

At time same time, one measures, in the same conditions, the same parameter for a plasma sample. One calculates then the relative filtration time by dividing the time required to filter the diluted blood sample by the time being necessary to filter the plasma sample. For each group the average and the standard error of the individual data obtained was reckoned. The statistical evaluation was obtained by means of the Dunnett test.

The results, being shown in the table 9, show that both in the red cells adhesivity test and also in the red cells filtration test, the compound according to the Example 1 has a moderate activity being totally proportional to that of the Pentoxyphyllin, but being almost the double of that ascertained after Buflomedil chlorhydrate administration, whose effect was very modest in both tests.

Table N° 9

Effects on the adhesivity and deformable capacity of the red cells in the rat.

| Treatment | Dose mM/kg/os | n° animals | Teitel method T1/2 min. M ± E.S. | Teitel method Δ % vs contr. | Schmidt-Schönbein method T1/2 min. M ± E.S. | Schmidt-Schönbein method Δ % vs contr. |
|---|---|---|---|---|---|---|
| Controls | --- | 10 | 3,64 ± 0,36 | --- | 0,457 ± 0,024 | --- |
| Compound as Ex. 1 | 3 x 0,5 | 10 | 2,26 ± 0,21 • | 37,9 | 0,712 ± 0,028 •• | 46,2 |
| pentossifillin | 3 x 0,5 | 10 | 2,18 ± 0,19 • | 40,1 | 0,744 ± 0,033 •• | 52,8 |
| Buflomedil | 3 x 0,5 | 10 | 2,90 ± 0,28 | 20,3 | 0,605 ± 0,026 • | 24,2 |

• $P < 0,05$ •• $P < 0,01$ vs controls (Dunnett test)

## Claims

1. Compound with the general formula (1) as follows

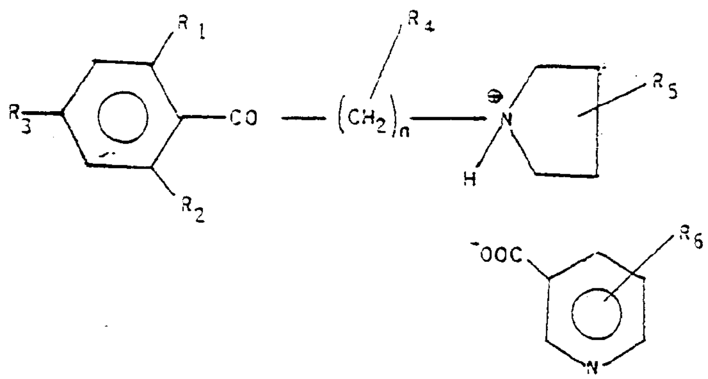

wherein $R_1$, $R_2$, $R_3$, which may be absent or may represent equal or different groups; the linear or branched-off $C_1$-$C_5$ alkyl group, or $C_1$-$C_5$ linear or branched-off alkoxy group, in "n" represents a whole number between 1 and 4, $R_4$ may be absent or may represent linear or branched-off $C_1$-$C_5$ alkyl group, or a halogen, or a nitrogen group; $R_5$ may be absent or may represent linear or branched-off $C_1$-$C_5$ alkyl groups, or otherwise linear or branched-off $C_1$-$C_5$ alkoxy groups, or a halogen, or a nitro-group; $R_6$ may be absent or may represent a linear or branched-off $C_1$-$C_5$ alkylic group, or a linear or branched-off $C_1$-$C_5$ alkoxy group, or a nitro group, or a halogen, or an ester group.

**2.** Hypolipaemizing active agent, with features as antispastic and vessels dilating and also antiaggregating agent consisting of a compound as claimed in the Claim 1.

**3.** Use of a compound such as it has been claimed In the Claim 1, for the preparation of a medicine having a vessels dilating, antispastic, hypolipaemizing effect and/or an antiaggregating effect.

**4.** Pharmaceutical compositions consisting of a therapeutically efficient quantity of a compound according with the formula (1) and a vehicle or carrier and also, or, a pharmaceutically tolerable excipient.

**5.** A compound as claimed in the Claim (4) in form of tablets having the following formula:
Example of formulation of gastroresistant tablets:
Compound according to the Example 1: 375,5 g
Cover solution
Eudragit L: 18,8 g
Peg 6000 FU: 1,8 g
Di-butylphtalate: 0,5 g
Di-methicone 2000 FU: 0,3 g
Talc FU: 3,6 g